# EUROPÄISCHE PATENTANMELDUNG

(11) **EP 3 622 943 A1**
(43) Veröffentlichungstag der Anmeldung: **18.03.2020**
(21) Anmeldenummer: 18196066.7
(22) Anmeldetag: 21.09.2018
(51) Int. Cl.: A61K 6/083, C08L 33/12, C08F 220/36

(54) **POLYMERISIERBARE WERKSTOFFE AUF BASIS VON DIMERISIERUNGSFÄHIGEN BENZALDEHYD-DERIVATEN**

(30) Priorität: 11.09.2018 EP 18193807
(71) Anmelder: Ivoclar Vivadent AG, 9494 Schaan (LI)
(72) Erfinder: MOSZNER, Norbert, 9495 Triesen (LI); LAMPARTH, Iris, 9472 Grabs (CH); BARNER-KOWOLLIK, Christopher, Kenmore, Queensland 4069 (AU); KRAPPITZ, Tim, Red Hill, Queensland 4059 (AU); FEIST, Florian, Auchenflower, Queensland 4066 (AU); JOHN, Hendrik, 9470 Buchs SG (CH)
(74) Vertreter: Uexküll & Stolberg

(57) **Zusammenfassung**

Die vorliegende Erfindung betrifft polymerisierbare und photochemisch vernetzbare Zusammensetzungen, die mindestens ein polymerisierbares Benzaldehyd-Derivat gemäß der allgemeinen Formel I enthalten 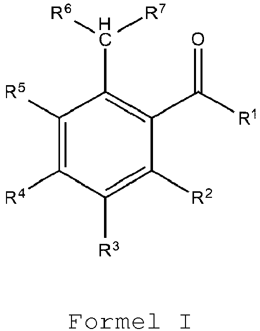 und die sich als Werkstoffe für technische und medizinische Anwendungen, beispielsweise in der Chirurgie oder Augenheilkunde, und insbesondere als Dentalwerkstoffe eignen.

## Beschreibung

Die vorliegende Erfindung betrifft polymerisierbare und photochemisch vernetzbare Zusammensetzungen auf der Basis von Benzaldehyd-Derivaten, die sich als Werkstoffe für technische und medizinische Anwendungen, beispielsweise in der Chirurgie oder Augenheilkunde, und insbesondere als Dentalwerkstoffe eignen.

Generative Fertigungsverfahren, bei denen aus computergestützten Konstruktionsdaten (CAD-Daten) dreidimensionale Bauteile schichtweise hergestellt werden, haben heutzutage in vielen technischen Bereichen und für die Herstellung von Medizinprodukten große Verbreitung gefunden. Dabei handelt es sich um Verfahren wie z.B. die Stereolithographie (SL), 3D-Printing, Ink-Jet-Printing (IJP), 3D-Plotting, Multijet Modelling (MJM) Solid-Freeform Fabrication (SFF) oder Laser Powder Forming (LPF), mit denen sich Modelle, Bau- und Formteile kostengünstig herstellen lassen. Derartige Verfahren werden auch als additive Verfahren bezeichnet.

Dentale Formteile werden meist aus einer flüssigen und härtbaren Mono- oder Oligomerzusammensetzung aufgebaut. Dafür verwendete Zusammensetzungen basieren in der Regel auf Mischungen von radikalisch polymerisierbaren (Meth)acrylaten oder kationisch polymerisierbaren Epoxid-, Vinylether- oder Oxetanzusammensetzungen. Dabei werden meist di- oder multifunktionelle Monomere verwendet, und es entstehen relativ spröde Netzwerkpolymere. Neben den polymerisierbaren Komponenten enthalten die Werkstoffe häufig einen oder mehrere Füllstoffe. Die polymerisierbaren Komponenten werden auch als Matrix oder Harz bezeichnet.

In der Dentalindustrie wird zur Herstellung von Korrekturschienen für die Orthodontie, sogenannten Alignern, mittels generativer Fertigungsverfahren zunächst ein Kiefer-/Zahn-Modell durch 3D-Druck hergestellt. Um die Korrekturschiene zu erhalten, wird anschließend eine spezielle Tiefziehfolie, die beispielsweise auf Polyurethan (PU), Polyethylenterephthalat (PET) oder Polypropylen (PP) basiert, über dieses Modell tiefgezogen. Danach wird das Modell verworfen. Es handelt sich hierbei um ein indirektes Verfahren zur Herstellung der Korrekturschienen. Die Korrektur der Zahnstellung erfolgt in mehreren Schritten. Nach Anwendung der ersten Korrekturschiene beim Patienten wird ein neues Modell der geänderten Zahnsituation auf die beschriebene Weise angefertigt und das Modell anschließend wieder verworfen. Da häufig bis zu 20 Behandlungsstufen erforderlich sind, um die gewünschte Zahnstellung zu erreichen, ist die Behandlung mit einem erheblichen Materialaufwand verbunden.

Materialien, die direkt durch generative Verfahren verarbeitet werden können, erreichen bisher nicht die für Korrekturschienen erforderlichen mechanischen und biokompatiblen Eigenschaften. Bekannten Werkstoffe, die annähernd geeignete mechanischen Eigenschaften ergeben, weisen eine so hohe Viskosität auf, dass sie nur bei hohen Temperaturen und nicht bei Raumtemperatur verarbeitet werden können.

Die WO 2013/104486 A1 offenbart Kleb- und Beschichtungsmassen, die mittels einer photoinduzierten Diels-Alder- oder Hetero-Diels-Alder-Reaktion vernetzbar sind. Die Materialen enthalten eine Komponente, die mindestens zwei dienophile Doppelbindungen aufweist, und eine zweite Komponente mit mindestens zwei Diengruppen-bildenden Funktionalitäten. Mindestens eine dieser Komponenten muss mehr als zwei Funktionalitäten aufweisen, um eine Vernetzung zu bewirken.

Die US 2017/0007362 A1 beschreibt vernetzte Polymere, die für kieferorthopädische Apparaturen geeignet sein sollen und in direkten Fertigungsverfahren eingesetzt werden können. Ferner offenbart sie lichtinduziert polymerisierbare Zusammensetzungen, aus denen sich die vernetzten Polymere herstellen lassen. Die Vernetzung erfolgt über multifunktionelle Vinylmonomere, wie z.B. Di(meth)acrylate oder Epoxy(meth)acrylate, oder über die Reaktion von funktionellen Gruppen, die Doppelbindungen aufweisen, mit Thiolen.

Der Erfindung liegt die Aufgabe zugrunde, Werkstoffe bereitzustellen, die sich für die stereolithographische Verarbeitung eignen und die im ausgehärteten Zustand mechanische Eigenschaften aufweisen, die für medizintechnische sowie für dentale Zwecke und insbesondere für dentale Korrekturschienen geeignet sind. Ferner sollen die Materialen eine für die genannten Anwendungen geeignete Biokompatibilität haben.

Außerdem sollen die Materialien nach der Härtung eine möglichst geringe Abnahme der Rückstellkräfte über einen möglichst langen Zeitraum zeigen. Angestrebt wird ein Rückgang von maximal 15% bis 20% über einen Zeitraum von 14 Tagen.

Zudem sollten die Werkstoffe nach der Härtung eine hohe Transparenz und eine geringe Eigenfärbung aufweisen, so dass Korrekturschienen hergestellt werden können, die möglichst unauffällig sind. Darüber hinaus sollen die Werkstoffe nur eine geringe Wasseraufnahme zeigen.

Die Aufgabe wird erfindungsgemäß durch Zusammensetzungen gelöst, die mindestens eine polymerisierbare Verbindung gemäß der allgemeinen Formel I enthalten, 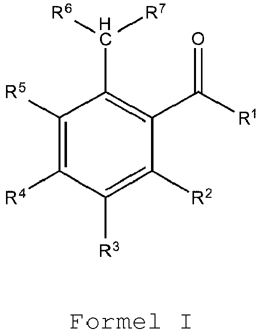 in der
- R¹: Wasserstoff, eine verzweigte oder unverzweigte, gesättigte oder ungesättigte C₁-C₁₄-Alkylgruppe, die durch O oder S unterbrochen sein kann, eine C₆-C₁₄-Arylgruppe oder eine C₄-C₁₄-Heteroarylgruppe ist, die N, O oder S enthalten kann, wobei die Alkyl-, Aryl- oder Heteroarylgruppen mit Thioether-, Amino-, Alkoxy- oder Alkylgruppen mit 1 bis 14 Kohlenstoffatomen oder C₆-C₁₄-Arylgruppen substituiert sein können und wobei die Substituenten miteinander verbrückt sein können,
- R²⁻⁵: jeweils unabhängig voneinander Wasserstoff, eine NH₂-Gruppe, eine verzweigte oder unverzweigte, gesättigte oder ungesättigte Thioether-, Amino-, Alkoxy- oder Alkylgruppe mit 1 bis 14 Kohlenstoffatomen, eine verzweigte oder unverzweigte, gesättigte oder ungesättigte C₇-C₁₅-Arylalkoxygruppe, vorzugsweise Benzyloxygruppe (Ph-CH₂-O-), oder eine C₆-C₁₄-Arylgruppe sind, die über 0 oder S angebunden sein kann, wobei R²⁻⁵ miteinander verbrückt sein können und R² und/oder R⁴ -X-SP-PG sind/ist,
- X: entfällt oder -O-, -CO-O- oder -O-CO- ist,
- SP: eine lineare oder verzweigte C₁-C₆-Alkylen-Gruppe ist, wobei die Alkylen-Gruppe durch O, S, -CO-O-, -O-CO- und/oder Phenylen(Ph) unterbrochen sein kann,
- PG: eine polymerisierbare Gruppe ist,
- R⁶⁻⁷: jeweils unabhängig voneinander Wasserstoff oder eine C₁-C₁₀-Alkylgruppe sind, die verzweigt oder unverzweigt, gesättigt oder ungesättigt sein kann.

Vorzugsweise ist die polymerisierbare Gruppe (PG) eine radikalisch oder kationisch polymerisierbare Gruppe, besonders bevorzugt eine radikalisch polymerisierbare Gruppe.

Bevorzugte radikalisch polymerisierbare Gruppen (rPG) sind Vinyl-, (Meth)acrylat- (-O-C(=O)-C(R⁸)=CH₂ mit R⁸ = H oder -CH₃) oder (Meth)acrylamid-Gruppen (-N(R⁹)-C(=O)-C(R¹⁰)=CH₂ mit R⁹ = H oder C₁-C₄-Alkyl und R¹⁰ = H oder -CH₃), besonders bevorzugt (Meth)acrylat-Gruppen.

Bevorzugte kationisch polymerisierbare Gruppen (kPG) sind Epoxid-, Oxetan- oder Vinylether-Gruppen, besonders bevorzugt Epoxid-Gruppen.

Bevorzugt sind Verbindungen, in denen
- R¹: Wasserstoff oder eine C₁-C₄-Alkylgruppe ist,
- R²⁻⁵: jeweils unabhängig voneinander Wasserstoff oder eine C₁-C₄-Alkoxy- oder C₁-C₄-Alkylgruppe sind, wobei R² und/oder R⁴ -X-SP-PG sind/ist,
- X: -O- ist,
- SP: eine lineare C₂-C₆-Alkylen-Gruppe ist, wobei die Alkylen-Gruppe durch O, -CO-O- und/oder Phenylen (Ph) unterbrochen sein kann, vorzugsweise eine C₂-C₄-Alkylen-Gruppe oder -CH₂-Ph-CO-O-CH₂CH₂- ist,
- PG: eine kationisch oder vorzugsweise radikalisch polymerisierbare Gruppe, insbesondere eine (Meth)acrylat-Gruppe oder eine Acrylamid-Gruppe ist,
- R⁶⁻⁷: beide Wasserstoff oder R⁶ = C₁-C₁₀-Alkyl und R⁷ = H sind.

Besonders bevorzugt sind Verbindungen, in denen
- R¹: Wasserstoff oder -CH₃ ist,
- R²⁻⁵: jeweils unabhängig voneinander Wasserstoff, -CH₃ oder -OCH₃ sind, wobei R² oder R⁴ -X-SP-PG ist,
- X: -O- ist,
- SP: eine lineare C₂-C₆-Alkylen-Gruppe ist, wobei die Alkylen-Gruppe durch O, -CO-O- und/oder Phenylen (Ph) unterbrochen sein kann, vorzugsweise eine C₂-C₄-Alkylen-Gruppe oder -CH₂-Ph-CO-O-CH₂CH₂- ist,
- PG: eine kationisch oder vorzugsweise radikalisch polymerisierbare Gruppe, insbesondere eine (Meth)acrylat-Gruppe oder eine Acrylamid-Gruppe ist,
- R⁶⁻⁷: jeweils Wasserstoff sind.

Ganz besonders bevorzugt sind Verbindungen, in denen
- R¹: Wasserstoff ist,
- R²⁻⁵: jeweils unabhängig voneinander Wasserstoff oder -CH₃ sind, wobei R² oder R⁴ -X-SP-PG ist,
- X: -O- ist,
- SP: eine lineare C₂-C₄-Alkylen-Gruppe ist, wobei die Alkylen-Gruppe durch O, -CO-O- und/oder Phenylen (Ph) unterbrochen sein kann, vorzugsweise eine C₂-Alkylen-Gruppe oder -CH₂-Ph-CO-O-CH₂CH₂- ist,
- PG: eine radikalisch polymerisierbare Gruppe, insbesondere eine (Meth)acrylat-Gruppe oder eine Acrylamid-Gruppe ist,
- R⁶⁻⁷: jeweils Wasserstoff sind.

Die erfindungsgemäßen Zusammensetzungen enthalten neben der Verbindung der Formel I vorzugsweise keine weiteren 1,3-Diene und keine Dienophile, die Cycloadditionsreaktionen eingehen.

Die Formel I erstreckt sich nur auf solche Verbindungen, die mit der chemischen Valenzlehre vereinbar sind. Der Hinweis, dass ein Rest z.B. durch ein oder mehrere O-Atome unterbrochen ist, ist so zu verstehen, dass diese Atome jeweils in die Kohlenstoffkette des Restes eingeschoben werden. Diese Atome sind damit beidseitig durch C-Atome begrenzt und können nicht endständig sein. C₁-Reste können nicht unterbrochen sein.

Die erfindungsgemäßen Benzaldehyd-Derivate der allgemeinen Formel I sind auf an sich bekannte Weise zugänglich und lassen sich nach bekannten Synthesemethoden herstellen. Gemäß einer bevorzugten Syntheseroute werden z.B. polymerisierbare o-Methylbenzaldehyd-Derivate (P-MBA), die über einen Spacer (SP) eine gebundene polymerisierbare Gruppe (PG) tragen, ausgehend von 2,3-Dimethylanisol (DMA) über 2-Methoxy-5-methylbenzaldehyd (MMBA) und 2-Hydroxy-5-methyl-benzaldehyd (HMBA) hergestellt:

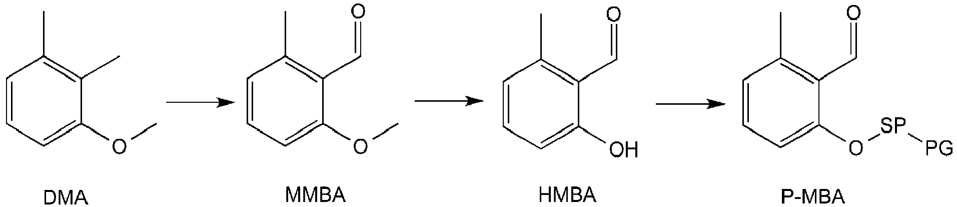

Das besonders bevorzugte 4-[(2-Formyl-3-methylphenoxy)-methyl]-benzoesäure-(2-methacryloyloxyethyl)-ester (PEMA) lässt sich vorzugsweise aus dem kommerziell zugänglichen 2,3-Dimethylanisol herstellen. Dabei erfolgt zunächst die Oxidation zum 2-Methoxy-6-methylbenzaldehyd in Gegenwart von Kaliumperoxodisulfat und anschließend die Abspaltung der Methoxy-Gruppe in Gegenwart von Aluminiumchlorid. Daraufhin wird das erhaltene 2-Hydroxy-6-methylbenzaldehyd mit 4-(Brommethyl)benzoesäuremethylester umgesetzt und der Ester verseift. Die freigesetzte 4-[(2-Formyl-3-methylphenoxy)-methyl]-benzoesäure wird abschließend mit Hydroxyethylmethacrylat zu PEMA verestert (Pauloehrl et al., Angew. Chem. Int. Ed. Engl. 51 (2012) 1071-1074; Klaus et al., Angew. Chem. Int. Ed. Engl. 55 (2016) 3817-3822):

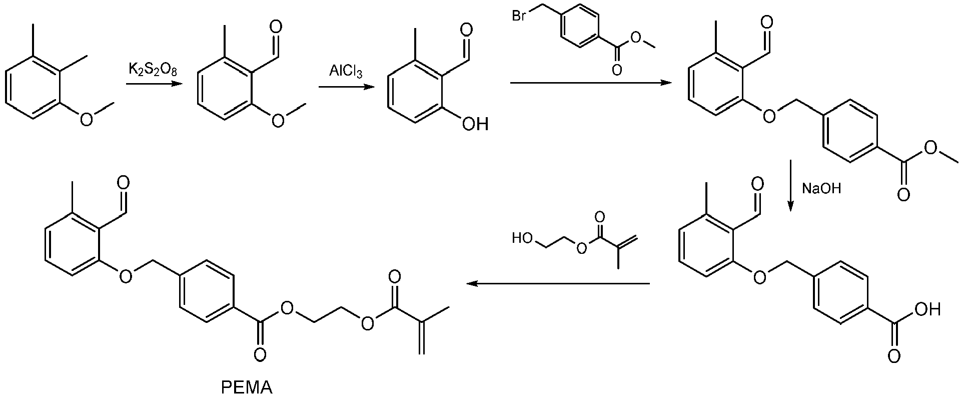

Bevorzugte Benzaldehyd-Derivate der allgemeinen Formel I sind:

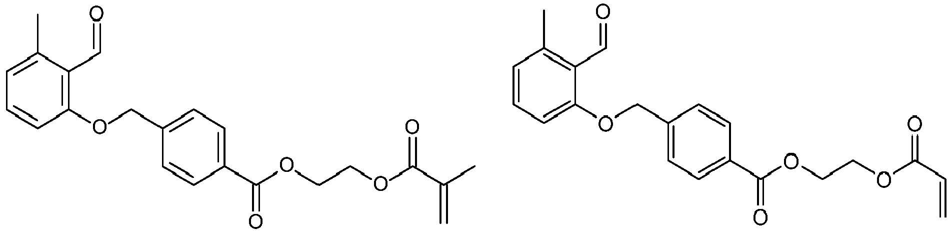

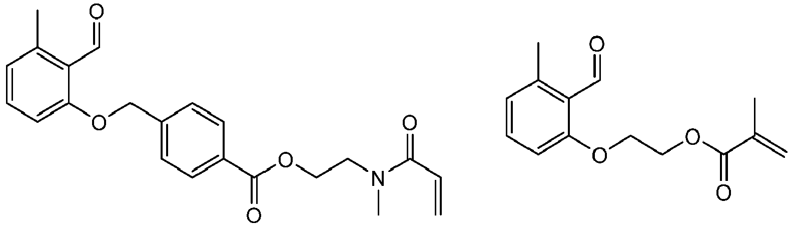

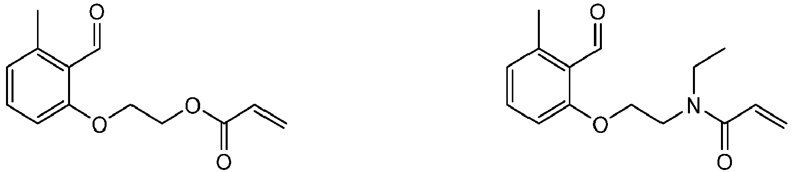

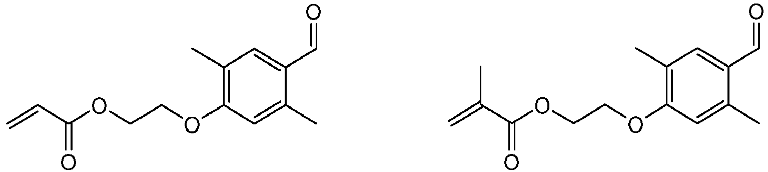

Ein besonders bevorzugtes Benzaldehyd-Derivat der allgemeinen Formel I ist:

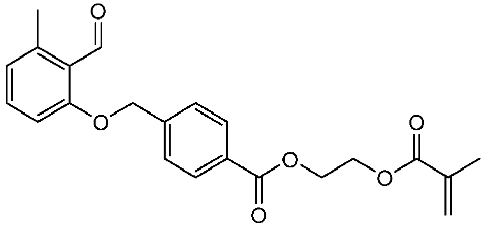

Die erfindungsgemäßen Benzaldehyd-Derivate können während oder nach der Polymerisation photochemisch vernetzt werden. Dazu werden sie mit UV-A-Licht bestrahlt, das eine Dimerisierung der Benzaldehyd-Derivate mittels einer Cycloadditionsreaktion bewirkt.

Die erfindungsgemäßen Benzaldehyd-Derivate können direkt, d.h. in monomerer Form, oder in oligomerer oder polymerer Form als Harzkomponente eingesetzt werden. Oligomere oder polymerer Formen lassen sich mit bekannten Methoden durch radikalische bzw. kationische Homo- oder Copolymerisation der Benzaldehyd-Derivate in Substanz oder Lösung herstellen. Die monomere Form der polymerisationsfähigen Benzaldehyd-Derivate ist bevorzugt.

Die monomeren, oligomeren und/oder polymeren Benzaldehyd-Derivate lassen sich als Werkstoffe zur Herstellung von Formkörpern durch generative Verfahren verwenden.

Vorzugsweise enthalten die Zusammensetzungen neben den erfindungsgemäßen Benzaldehyd-Derivaten der Formel I ein oder mehrere polymerisationsfähige Comonomere, wobei als Comonomere 1,3-Diene und Dienophile, die Cycloadditionsreaktionen eingehen können, vorzugsweise ausgeschlossen sind. Bevorzugte Comonomere sind kationisch oder vorzugsweise radikalisch polymerisierbare Monomere.

Als Dentalmaterialien, insbesondere zur Herstellung von Zahnspangen oder Schienen zur Korrektur von Zahnfehlstellungen, sind Werkstoffe bevorzugt, die als radikalisch polymerisierbares Monomer mindestens ein mono- oder multifunktionelles (Meth)acrylat enthalten. Unter monofunktionellen (Meth)-acrylaten werden Verbindungen mit einer, unter multifunktionellen (Meth)acrylaten Verbindungen mit zwei oder mehr, vorzugsweise 2 bis 4 radikalisch polymerisierbaren Gruppen verstanden. Die mono- oder multifunktionellen (Meth)acrylate können weitere funktionelle Gruppen, wie z.B. Hydroxy-, Ester-Silyl- oder Ureido-Gruppen enthalten. Über die Struktur der radikalisch polymerisierbaren (Meth)acrylat-Comonomere lassen sich die Flexibilität und die Eigenschaften der gehärteten Materialien gezielt beeinflussen.

Bevorzugte monofunktionelle (Meth)acrylate sind Methyl-, Ethyl-, n-Propyl, n-Butyl-, 2-Ethylhexyl-, Cyclohexyl-, Benzyl-, Tetrahydrofurfuryl-, Isobornyl- oder Lauryl(meth)acrylat sowie p-Cumyl-phenoxyethylenglycol(meth)acrylat oder 2-(2-Biphenyloxy)-ethyl(meth)acrylat und Mischungen davon. Besonders bevorzugte monofunktionelle (Meth)acrylate sind Methylacrylat (MA), Ethylacrylat (EA), Methylmethacrylat (MMA), Butylmethacrylat (BMA), Tetrahydrofurfurylmethacrylat (THFMA), Isobornylmethacrylat (IBOMA) und Mischungen davon. Ganz besonders bevorzugt sind Methylacrylat (MA), Methylmethacrylat (MMA), Butylmethacrylat (BMA), Tetrahydrofurfurylmethacrylat (THFMA), Isobornylmethacrylat (IBOMA) und Mischungen davon.

Bevorzugte monofunktionelle (Meth)acrylate mit zusätzlichen funktionellen Gruppen sind Hydroxyalkyl-, Alkyloxycarbonylalkyl-, Silyl- und Ureido(meth)acrylate, wie z.B. 2-Hydroxyethyl-, 2-Hydroxypropyl-, 2-Acetoxyethyl-, 3-Trimethylsilylpropyl- und 2-Ureido-4-[1H]-pyrimidon(meth)acrylat, sowie säuregruppenhaltige (Meth)acrylate, wie z.B. (Meth)acrylsäure, 2-(Hydroxymethyl)acrylsäure, 4-(Meth)acryloyloxyethyltrimellitsäure, 10-Methacryloyloxydecylmalonsäure, 2-Methacryloyloxyethylphosphonsäure, oder 2-[4-(Dihydroxyphosphoryl)-2-oxa-butyl]-acrylsäure, und Mischungen davon. Besonders bevorzugte monofunktionelle (Meth)acrylate mit zusätzlichen funktionellen Gruppen sind 2-Hydroxyethyl- und 2-Hydroxypropyl(meth)acrylat, 2-Ureido-4-[1H]-pyrimidon(meth)acrylat und Mischungen davon. Ein ganz besonders bevorzugtes monofunktionelle (Meth)acrylat mit zusätzlichen funktionellen Gruppen ist 2-Ureido-4-[1H]-pyrimidon(meth)acrylat.

Bevorzugte multifunktionelle (Meth)acrylate sind Bisphenol-A-di(meth)acrylat, wie z.B. 2,2-Bis[4-(2-hydroxy-3-(meth)acryloyloxypropyl)phenyl]propan (Bis-G(M)A; ein Additionsprodukt aus (Meth)acrylsäure und Bisphenol-A-diglycidylether), ethoxy- oder propoxyliertes Bisphenol-A-di(meth)acrylat, wie z.B. 2-[4-(2-Methacryloyloxyethoxyethoxy)phenyl]-2-[4-(2-methacryloyloxyethoxy)phenyl]-propan) (SR-348c, Firma Sartomer,; enthält 3 Ethoxygruppen), 2,2-Bis[4-(2-(meth)acryloxypropoxy)phenyl]propan, 1,6-Bis-[2-(meth)acryloyloxyethoxycarbonylamino]-2,2,4-trimethylhexan (UD(M)A; ein Additionsprodukt aus 2-Hydroxyethyl(meth)acrylat und 2,2,4-Trimethylhexamethylendiisocyanat), Di-, Tri- oder Tetraethylenglycoldi(meth)acrylat, Trimethylolpropantri(meth)acrylat, Pentaerythrittetra(meth)-acrylat, Glycerindi- und -tri(meth)acrylat, 1,4-Butandiol-di(meth)acrylat, 1,10-Decandioldi(meth)acrylat (D₃(M)A), 1,12-Dodecandioldi(meth)acrylat und Mischungen davon. Besonders bevorzugte multifunktionelle (Meth)acrylate sind Di-, Tri- oder Tetraethylenglycoldi(meth)acrylat, Glycerindi- und -tri(meth)-acrylat, 1,4-Butandioldi(meth)acrylat, 1,10-Decandioldi(meth)-acrylat (D₃(M)A), 1,12-Dodecandioldi(meth)acrylat und Mischungen davon. Ganz besonders bevorzugte multifunktionelle (Meth)acrylate sind Di-, Tri- oder Tetraethylenglycoldi(meth)-acrylat, 1,4-Butandioldi(meth)acrylat, 1,10-Decandioldi(meth)-acrylat (D₃(M)A) und Mischungen davon.

Bevorzugte (Meth)acrylamide sind flüssige tertiäre Acrylamide, wie z.B. N,N-Dimethylacrylamid, N,N-Diethylacrylamid, N,N-Di-isopropylacrylamid, N-(2-Hydroxyethyl)-N-methyl-acrylamid oder N,N'-Diethyl-1,3-bis(acrylamido)-propan, flüssige sekundäre Methacrylamide, wie z.B. 2-Ureido-4[1H]-pyrimidonmethacrylamid (UPyMAA), und Mischungen davon. Besonders bevorzugte (Meth)-acrylamide sind N,N-Dimethylacrylamid, N,N-Diethylacrylamid, N,N'-Diethyl-1,3-bis(acrylamido)-propan, 2-Ureido-4[1H]-pyrimidonmethacrylamid (UPyMAA) und Mischungen davon. Ganz besonders bevorzugte (Meth)acrylamide sind N,N-Dimethylacrylamid, N,N-Diethylacrylamid, 2-Ureido-4[1H]-pyrimidonmethacrylamid (UPyMAA) und Mischungen davon.

Bevorzugte kationische polymerisierbare Monomere sind kationisch ringöffnend polymerisierbare Monomere, insbesondere Glycidylether, cycloaliphatische Epoxide und Oxetane, wie z.B. Phenylglycidyl- Benzylglycidyl oder Bisphenol-A-diglycidylether, Cyclohexenoxid, 3,4-Epoxy-cyclohexylmethyl-3,4-epoxycyclohexancarboxylat, Bis(3,4-epoxycyclohexylmethyl)adipat, Vinylcyclohexendioxid, 3-Ethyl-3-hydroxymethyloxetan, 1,10-Decandiyl-bis-(oxymethylen)-bis-(3-ethyloxetan) oder 3,3-(4-Xylylendioxy)-bis-(methyl-3-ethyloxetan) und Mischungen davon. Besonders bevorzugte kationisch polymerisierbare Monomere sind 3,4-Epoxy-cyclohexylmethyl-3,4-epoxycyclohexancarboxylat, Bis(3,4-epoxycyclohexylmethyl)adipat, 3-Ethyl-3-hydroxymethyl-oxetan, 1,10-Decandiyl-bis-(oxymethylen)-bis-(3-ethyloxetan) und Mischungen davon. Ganz besonders bevorzugte kationisch polymerisierbare Monomere sind 3,4-Epoxy-cyclohexylmethyl-3,4-epoxycyclohexancarboxylat, 3-Ethyl-3-hydroxymethyloxetan und Mischungen davon.

Die erfindungsgemäßen Zusammensetzungen können entweder ein- oder zweistufig ausgehärtet werden. Vorzugsweise erfolgt die Aushärtung bei der stereolithographischen Verarbeitung der Werkstoffe. Die einstufige Aushärtung erfolgt vorzugsweise durch UV-A-Licht, besonders bevorzugt mit einer Wellenlänge im Bereich von 320 bis 380 nm. Hierzu enthalten die Zusammensetzungen vorzugsweise zusätzlich einen UV-A-Photoinitiator.

Durch die Bestrahlung mit UV-A-Licht wird die Polymerisation der erfindungsgemäßen Benzaldehyd-Derivate der Formel I mit den ggf. vorhandenen Comonomeren initiiert. Gleichzeitig wird durch die UV-A-Bestrahlung eine Vernetzung des Werkstoffs bewirkt.

Die Vernetzung erfolgt über die erfindungsgemäßen Benzaldehyd-Derivate. Die Bestrahlung z.B. des o-Methylbenzaldehyds MMBA mit UV-A-Licht führt zur intermediären Bildung eines Enols, eines o-Quinodimethan-Derivats (5,6-Bis(methylen)-1,3-cyclohexadien-Derivats), das mit einem elektronenarmen Dienophil, z.B. Maleiimid oder Fumarsäureester, zu einem entsprechenden Diels-Alder-Addukt reagieren kann. Fehlt ein geeignetes Dienophil, können zwei o-Quinodimethan-Gruppen in einer Cycloaddition dimerisieren und so eine Vernetzung von zwei Polymerketten bewirken:

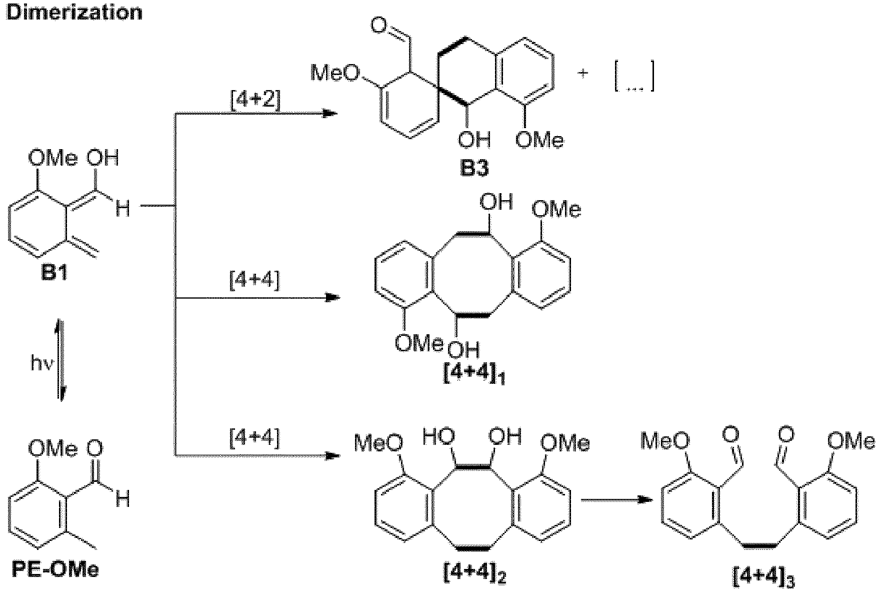

Es wurde gefunden, dass sich diese Reaktion vorteilhaft zur Herstellung von Formkörpern durch generative Verfahren ausnutzen lässt. Ein Vorteil der erfindungsgemäßen Benzaldehyd-Derivate ist, dass sich der Grad der Vernetzung und damit die Flexibilität der Polymernetzwerke durch die Dauer der Bestrahlung gezielt einstellen lässt.

Die Vernetzungsdichte der Polymernetzwerke kann dabei während und nach der Verarbeitung durch die Bestrahlungsdauer gesteuert werden. Auf diese Weise können bei gegebener Ausgangszusammensetzung Werkstoffe mit unterschiedlichem Vernetzungsgrad und damit mit unterschiedlichen mechanischen Eigenschaften erhalten werden. Dem entgegen ist die Vernetzungsdichte bei der Verwendung von multifunktionellen (Meth)acrylaten als Vernetzer durch deren Anteil in der Zusammensetzung vorgegeben, sodass für unterschiedliche Vernetzungsdichten jeweils eine andere Zusammensetzung vorgehalten werden muss. Die Kombination von erfindungsgemäßen Benzaldehyd-Derivaten und multifunktionellen (Meth)acrylaten ermöglicht eine grundlegende Vernetzung über den Anteil an multifunktionellen (Meth)acrylaten, die durch die photoinduzierte Dimerisierung der Benzaldehyd-Derivate gezielt variiert werden kann. In Kombinationen von erfindungsgemäßen Benzaldehyd-Derivaten und multifunktionellen (Meth)acrylaten sollte der Anteil an multifunktionellen (Meth)acrylaten vorzugsweise nicht größer als 20 Gew.-%, besonders bevorzugt nicht größer als 15 Gew.-% und ganz besonders bevorzugt nicht größer als 10 Gew.-% sein, jeweils bezogen auf die Gesamtmasse der Zusammensetzung.

Außerdem ist ein weiterer Vorteil der erfindungsgemäßen Benzaldehyd-Derivate, dass die Vernetzungsdichte der Polymernetzwerke im Erzeugnis durch die Bestrahlung lokal variiert werden kann, sodass Erzeugnisse mit unterschiedlichem Vernetzungsgrad in verschiedenen Bereichen des Erzeugnisses hergestellt werden können. Weiterhin kann die Vernetzung auch erst am Ende der Fertigstellung der Formteile erfolgen.

Zur zweistufigen Aushärtung wird den Zusammensetzungen zusätzlich ein Photoinitiator für den sichtbaren Bereich, ein thermischer Initiator oder ein Redoxinitiator zugesetzt. Bei der Bestrahlung mit sichtbarem Licht oder bei der Verwendung eines thermischen Initiators oder Redoxinitiators findet in der ersten Stufe zunächst eine radikalische oder kationische Polymerisation der Benzaldehyd-Derivate und der ggf. vorhandenen Comonomere statt. In der zweiten Stufe erfolgt eine Bestrahlung mit UV-A-Licht, was zur Vernetzung der in der ersten Stufe gebildeten Polymerketten durch Dimerisierung der in den Ketten vorhandenen Benzaldehyd-Derivat-Gruppen führt.

Die Netzwerkstruktur und die Eigenschaften der gebildeten Netzwerkpolymere können über den Gehalt an erfindungsgemäßen Benzaldehyd-Derivaten der Formel I sowie die Bestrahlungsdauer gesteuert werden. Die maximale Vernetzungsdichte nimmt mit dem Gehalt an erfindungsgemäßen Benzaldehyd-Derivaten der Formel I zu, wobei die Vernetzungsdichte im Einzelfall über die Bestrahlungsdauer mit UV-A-Licht gesteuert werden kann. Eine längere Bestrahlungsdauer bewirkt einen höheren Dimerisierungs- und damit einen höheren Vernetzungsgrad.

Bevorzugte Photoinitiatoren für den UV-A-Bereich (320 bis 380 nm) sind Benzophenon, Benzoin, 2,2-Dimethoxy-1,2-diphenylethan-1-on (Irgacure® 651), 1-Hydroxycyclohexylphenylketone (Irgacure® 184), 2-Hydroxy-2-methyl-1-phenylpropanon (Irgacure® 1173) sowie Acyl- und Bisacylphosphinoxide, wie z.B. die kommerziell erhältlichen Verbindungen 2,4,6-Trimethylbenzoyldiphenylphosphinoxid (Lucerin® TPO), Ethyl-(2,4,6-trimethylbenzoyl)phenylphosphinat (Irgacure® TPO-L) und Bis(2,4,6-trimethylbenzoyl)phenylphosphinoxid (Irgacure® 819), und Mischungen davon. Besonders bevorzugt sind Campherchinon (CC), 2,2-Dimethoxy-2-phenyl-acetophenon, Bis(2,4,6-trimethylbenzoyl)-phenylphosphinoxid (Irgacure® 819) 2,4,6-Trimethylbenzoyldiphenylphosphinoxid (Lucerin® TPO), Ethyl-(2,4,6-trimethylbenzoyl)phenylphosphinat (Irgacure® TPO-L) und Mischungen davon.

Bevorzugte Photoinitiatoren für den sichtbaren Bereich (380 bis 780 nm) sind α-Diketone oder deren Derivate, wie 9,10-Phenanthrenchinon, 1-Phenyl-propan-1,2-dion, Diacetyl oder 4,4'-Dichlorbenzil. Vorzugsweise eignen sich auch Kombinationen von α-Diketonen mit Aminen als Reduktionsmittel, wie z.B. 4-(Dimethylamino)-benzoesäureester (EDMAB), N,N-Dimethylaminoethylmethacrylat, N,N-Dimethyl-sym.-xylidin oder Triethanolamin. Geeignete Norrish-Typ-I-Photoinitiatoren für den sichtbaren Bereich sind Monoacyltrialkyl-, Diacyldialkyl- oder Tetraacylgermanium-Verbindungen sowie Tetraacylstannane, wie z.B. Benzoyltrimethylgerman, Dibenzoyldiethylgerman, Bis(4-methoxybenzoyl)diethylgerman (MBDEGe), Tetrakis(2-methyl-benzoyl)german oder Tetrakis(mesitoyl)stannan. Besonders bevorzugt ist Bis(4-methoxybenzoyl)diethylgermanium (MBDEGe).

Außerdem lassen sich als Initiatoren für den sichtbaren Bereich bevorzugt auch Mischungen der genannten Photoinitiatoren mit Aminen als Beschleuniger einsetzen. Besonders bevorzugt ist die Kombination von Campherchinon (CC) und 4-Dimethylaminobenzoesäureethylester (EDMAB).

Bevorzugte Photoinitiatoren für die kationische Polymerisation sind aromatische Diaryliodonium- oder Triarylsulfoniumsalze, z.B. die kommerziell zugänglichen Verbindungen 4-Octyloxyphenyl-phenyl-iodoniumhexafluoroantimonat oder -phosphat, Isopropylphenyl-methylphenyl-iodoniumtetrakis(pentafluorphenyl)-borat oder Triarylsulfoniumhexafluoroantimonat (CYRACURE® UVI 6976). Besonders bevorzugt sind 4-Octyloxyphenyl-phenyliodoniumhexafluoroantimonat oder -phosphat und Isopropylphenyl-methylphenyl-iodoniumtetrakis(pentafluorphenyl)borat. Diese sind im Wellenlängenbereich von 320 bis 400 nm aktiv.

Bevorzugte thermische Initiatoren für die radikalische Polymerisation sind Azoverbindungen, wie z.B. 2,2'-Azobis-(isobutyronitril) (AIBN) oder Azobis-(4-cyanovaleriansäure), Peroxide, wie z.B. Dibenzoylperoxid, Dilauroylperoxid, *tert-*Butylperoctoat, tert-Butylperbenzoat oder Di-(tert-butyl)-peroxid, Redox-Initiatorkombinationen, wie z.B. Kombinationen von Benzoylperoxid mit Aminen, wie z.B. N,N-Dimethyl-p-toluidin N,N-Dihydroxyethyl-p-toluidin, p-Dimethylaminobenzoesäureethylester oder N,N-Dimethyl-sym.-xylidin, Kombinationen von anorganischen Peroxiden, wie z.B. Kalium- und Ammoniumperoxodisulfat, mit Reduktionsmitteln, wie z.B. Sulfit, Hydrogensulfit, Thiosulfat, Sulfinsäuren, Aminen, Endiolen oder Fe-(II)-Salzen, oder Redoxsysteme bestehend aus organischen Peroxiden bzw. Hydroperoxiden und Reduktionsmitteln, wie z.B. Ascorbinsäure, Barbituraten, Thioharnstoffen oder Sulfinsäuren. Die erfindungsgemäßen Zusammensetzungen können vorzugsweise einen oder mehrere thermische Initiatoren enthalten.

Als Redoxinitiatoren sind Verbindungen von Übergangsmetallen bevorzugt, die mindestens zwei stabile Wertigkeitsstufen aufweisen. Das sind vor allem Verbindungen der Elemente Kupfer, Eisen, Vanadium, Nickel oder Kobalt, wobei Kupferverbindungen besonders bevorzugt sind und diese bevorzugt als gut organolösliche Verbindungen, wie z.B. als Acetylacetonat, Naphthenat oder 2-Ethyl-hexanoat eingesetzt werden.

Die erfindungsgemäßen Zusammensetzungen enthalten weiterhin vorzugsweise einen oder mehrere organische oder vorzugsweise anorganische Füllstoffe, wobei partikuläre Füllstoffe bevorzugt sind. Bevorzugte anorganische partikuläre Füllstoffe sind Oxide, wie SiO₂, ZrO₂ und TiO₂ oder Mischoxide aus SiO₂, ZrO₂, ZnO und/oder TiO₂ mit einer Partikelgröße von 0,005 bis 1,0 µm, nanopartikuläre oder mikrofeine Füllstoffe mit einer Partikelgröße von 5 bis 300 nm, wie pyrogene Kieselsäure oder Fällungskieselsäure, und röntgenopake Füllstoffe, wie Ytterbiumtrifluorid, Tantal(V)-oxid, Bariumsulfat oder Mischoxide von SiO₂ mit Ytterbium(III)-oxid oder Tantal(V)-oxid, mit einer Partikelgröße von 0,2 bis 5 µm.

Wenn nicht anders angegeben, handelt es sich bei allen Partikelgrößen um gewichtsmittlere Partikelgrößen, bei denen die Partikelgrößenbestimmung mittels statischer Lichtstreuung erfolgt, vorzugsweise mit einem statischen Laserstreuungs-Partikelgrößen-Analysator LA-960 (Horiba, Japan). Hierbei werden als Lichtquellen eine Laser-Diode mit einer Wellenlänge von 655 nm und eine LED mit einer Wellenlänge von 405 nm in einem Messbereich von 0,1 bis 1000 µm verwendet. Der Einsatz von zwei Lichtquellen mit unterschiedlichen Wellenlängen ermöglicht die Vermessung der gesamten Partikelgrößenverteilung einer Probe in nur einem Messungsdurchgang, wobei die Messung als Nassmessung durchgeführt wird. Hierzu wird eine 0,1 bis 0,5%ige wässrige Dispersion des Füllstoffs hergestellt und deren Streulicht in einer Durchflusszelle gemessen. Die Streulichtanalyse zur Berechnung von Partikelgröße und Partikelgrößenverteilung erfolgt gemäß der Mie-Theorie nach DIN/ISO 13320.

Die Messung der Partikelgröße im Bereich von 5 nm bis 0,1 µm erfolgt vorzugsweise durch Dynamische Lichtstreuung (DLS) von wässrigen Partikeldispersionen mit einem ALV / CGS-3 Compact Goniometer (ALV-Laser Vertriebsgesellschaft, Langen, Deutschland) mit einem He-Ne-Laser mit einer Wellenlänge von 633 nm, bei einem Streuwinkel von 90° bei 25°C.

Füllstoffe werden nach der Partikelgröße unterteilt in Makrofüller und Mikrofüller. Makrofüller werden z.B. durch Mahlen von Quarz, röntgenopaken Gläsern, Borosilikaten oder von Keramik gewonnen, sind vorzugsweise rein anorganischer Natur und bestehen meist aus splitterförmigen Teilen, die häufig eine mittlere Partikelgröße von 0,3 bis 15 µm aufweisen. Als Mikrofüller werden vorzugsweise pyrogenes SiO₂ oder Fällungskieselsäure eingesetzt oder auch Mischoxide, z.B. SiO₂-ZrO₂, die durch hydrolytische Cokondensation von Metallalkoxiden zugänglich sind. Die Mikrofüller weisen vorzugsweise eine mittlere Partikelgröße von 5 bis 100 nm auf. Füllstoffe mit geringer Partikelgröße haben eine größere Verdickungswirkung.

Die Füllstoffe sind vorzugsweise oberflächenmodifiziert, besonders bevorzugt durch Silanisierung, ganz besonders bevorzugt durch radikalisch polymerisierbare Silane, insbesondere mit 3-Methacryloyloxypropyltrimethoxysilan. Zur Oberflächenmodifizierung von nicht-silikatischen Füllstoffen, z.B. von ZrO₂ oder TiO₂, können auch funktionalisierte saure Phosphate, wie z.B. 10-Methacryloyloxydecyldihydrogenphosphat eingesetzt werden.

Die erfindungsgemäßen Zusammensetzungen können vorteilhaft weitere Additive enthalten, vor allem Stabilisatoren, Farbmittel, optische Aufheller, Weichmacher oder UV-Absorber.

Die erfindungsgemäßen Werkstoffe enthalten vorzugsweise die folgenden Komponenten:
a) 0,1 bis 50 Gew.-%, bevorzugt 1 bis 40 Gew.-% und besonders bevorzugt 1 bis 20 Gew.-% mindestens eines Benzaldehyd-Derivats der Formel I,
b) 30 bis 99 Gew.-%, bevorzugt 40 bis 95 Gew.-% und besonders bevorzugt 50 bis 95 Gew.-% mindestens eines weiteren monofunktionellen radikalisch oder kationisch polymerisierbaren Monomers,
c) 0 bis 20 Gew.-%, bevorzugt 0 bis 15 Gew.-% und besonders bevorzugt 0 bis 10 Gew.-% mindestens eines multifunktionellen radikalisch oder kationisch polymerisierbaren Monomers,
d) 0,001 bis 5,0 Gew.-%, bevorzugt 0,01 bis 3,0 Gew.-% und besonders bevorzugt 0,1 bis 1,0 Gew.-% mindestens eines Initiators, insbesondere eines Photoinitiators,
e) 0 bis 5,0 Gew.-%, bevorzugt 0 bis 3,0 Gew.-% und besonders bevorzugt 0 bis 1,5 Gew.-% Additiv(e).

Die erfindungsgemäßen Werkstoffe für generative Verfahren enthalten vorzugsweise die folgenden Komponenten:
a) besonders bevorzugt 5,0 bis 20 Gew.-% mindestens eines Benzaldehyd-Derivats der Formel I,
b) besonders bevorzugt 40 bis 94 Gew.-% mindestens eines weiteren monofunktionellen radikalisch oder kationisch polymerisierbaren Monomers,
c) besonders bevorzugt 0 bis 10 Gew.-% mindestens eines multifunktionellen radikalisch oder kationisch polymerisierbaren Monomers,
d) 0,1 bis 1,0 Gew.-% mindestens eines Initiators, insbesondere eines Photoinitiators,
e) besonders bevorzugt 0 bis 3,0 Gew.-% Additiv(e).
f) besonders bevorzugt 0 bis 40 Gew.-% Füllstoff(e).
Die erfindungsgemäßen Werkstoffe für Medizinprodukte enthalten vorzugsweise die folgenden Komponenten:
a) besonders bevorzugt 3,0 bis 10 Gew.-% mindestens eines Benzaldehyd-Derivats der Formel I,
b) besonders bevorzugt 40 bis 95 Gew.-% mindestens eines weiteren monofunktionellen radikalisch oder kationisch polymerisierbaren Monomers,
c) besonders bevorzugt 0 bis 15 Gew.-% mindestens eines multifunktionellen radikalisch oder kationisch polymerisierbaren Monomers,
d) 0,1 bis 1,0 Gew.-% mindestens eines Initiators, insbesondere eines Photoinitiators,
e) besonders bevorzugt 0,1 bis 3,0 Gew.-% Additiv(e).

Außerdem können die erfindungsgemäßen Zusammensetzungen einen oder mehrere Füllstoffe enthalten. Bevorzugt sind Zusammensetzungen, die 0 bis 50 Gew.-%, besonders bevorzugt 0 bis 40 Gew.-% und ganz besonders bevorzugt 0 bis 20 Gew.-% mindestens eines Füllstoffs enthalten. Zusammensetzungen ohne Füllstoffe sind besonders bevorzugt.

Alle Prozentangaben hierin sind in Gewichtsprozent und beziehen sich auf die Gesamtmasse des Werkstoffs, wenn nicht anders angegeben.

Besonders bevorzugt sind Zusammensetzungen, die aus den genannten Stoffen bestehen. Weiterhin sind solche Zusammensetzungen bevorzugt, bei denen die einzelnen Komponenten jeweils aus den oben genannten bevorzugten und besonders bevorzugten Stoffen ausgewählt sind.

Die erfindungsgemäßen Zusammensetzungen lassen sich durch stereolithographische Verfahren verarbeiten. Es wurde überraschenderweise gefunden, dass erfindungsgemäße Werkstoffe, die Benzaldehyd-Derivate der Formel I enthalten, nach der Härtung und Vernetzung vergleichbare mechanische Eigenschaften wie bekannte Tiefziehfolien auf Basis von Polyurethanen aufweisen. Typische Werte für derartige Folien sind ein E-Modul E_{red} von 0,5 bis 2,0 GPa und eine Härte von 30 bis 80 MPa. Die erfindungsgemäßen Materialien weisen nach Polymerisation und Vernetzung ein vergleichbares reduziertes Elastizitäts-Modul Eᵣ und eine vergleichbare Härte auf. Die erfindungsgemäßen Werkstoffe weisen bevorzugt ein reduziertes E-Modul Eᵣ von 0,30 bis 7,00 GPa, besonders bevorzugt 0,70 bis 6,00 GPa und ganz besonders bevorzugt von 0,90 bis 5,80 GPa, und eine Härte von 30 bis 500 MPa, besonders bevorzugt 40 bis 400 MPa und ganz besonders bevorzugt 50 bis 350 MPa auf.

Die mechanischen Eigenschaften (reduzierter E-Modul Eᵣ und Härte) der ausgehärteten Proben werden vorzugsweise mittels Nanoindentation (instrumentierter Eindringversuch) unter Verwendung eines Hysitron TL950 Nanoindentor (Bruker) bestimmt. Die ladungskontrollierten Messungen mit einer Berkovich-Spitze werden bei 2000 µN maximaler Ladekraft mit einer Ladezeit von 1 s, einem Ladesegment von 2 s sowie einer Lastwegnahme von 1 s durchgeführt. Die Analyse erfolgt unter Verwendung der Oliver- und Pharr-Methode (W. C. Oliver, G.M. Pharr, J. Mater In: Res. 7, 1992, S. 1564). Es wird mit Messmethoden analog zu B. Sandmann, B. Happ, J. Vitz, R. M. Paulus, M. D. Hager, P. Burtscher, N. Moszner, U. S. Schubert, Macromol. Chem. Phys. 2014, 215, 1603.) gemessen, die z.B. aus der Biegeprüfung vergleichbare E-Modulwerte für Dimethacrylatphotopolymerisate ergeben.

Die erfindungsgemäßen Werkstoffe eigenen sich besonders zur Herstellung von Formkörpern für dentale aber auch für nicht dentale Zwecke z.B. durch Gießen, Pressen und insbesondere durch generative Verfahren wie den 3D-Druck.

Die erfindungsgemäßen Werkstoffe eigenen sich weiterhin als medizintechnische Werkstoffe für die Chirurgie, wie z.B. als Werkstoffe zur Herstellung von Medizinprodukten wie Implantaten für Gehörprothesen, Knorpel- oder Knochenersatzteilen, und für die Augenheilkunde, wie z.B. als Werkstoffe zur Herstellung von Intraokularlinsen, die z.B. als künstliche Linsen für das Auge nach Entfernung der natürlichen Linse bei der Operation eines Grauen Stars verwendet werden, und insbesondere zur Herstellung von Dentalprothesen und Apparaturen für die Orthodontie. Die erfindungsgemäßen Werkstoffe eigenen sich ganz besondere als Dentalwerkstoffe zur Herstellung von Apparaturen für die Orthodontie wie Zahnspangen oder Schienen bzw. Schablonen zur Korrektur von Zahnfehlstellungen, insbesondere durch generative Verfahren.

Die Erfindung betrifft außerdem die Verwendung von Benzaldehyden der Formel I zur Herstellung von medizintechnischen Werkstoffen, insbesondere Dentalwerkstoffen.

Die Erfindung wird im Folgenden anhand von Beispielen näher erläutert.

### Ausführungsbeispiele

### Beispiel 1:

### 4-[(2-Formyl-3-methylphenoxy)-methyl]-benzoesäure-(2-meth-acryloyloxyethyl)-ester (PEMA)

### 1. Stufe: 2-Methoxy-6-methylbenzaldehyd (MMBA)

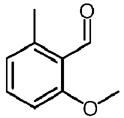

Ein Gemisch aus Kupfer(II)sulfat-Pentahydrat (18,14 g, 72,7 mmol) und Kaliumperoxodisulfat (59,81 g, 0,22 mol) in Wasser (700 ml) wurde mit einer Lösung von 2,3-Dimethylanisol (10,00 g, 73,4 mmol) in Acetonitril (500 ml) versetzt und 30 min unter Rückfluss erhitzt. Nach dem Abkühlen wurden Wasser (100 ml) und Dichlormethan (150 ml) zugegeben und die Phasen getrennt. Die wässrige Phase wurde mit Dichlormethan (2x 200 ml) extrahiert. Die vereinigten organischen Phasen wurden über wasserfreiem Natriumsulfat getrocknet, filtriert und am Rotationsverdampfer eingeengt. Das braune Öl wurde mit n-Heptan/Ethylacetat 4:1 (200 ml) versetzt und über Kieselgel filtriert. Das Filtrat wurde am Rotationsverdampfer eingeengt. Man erhielt 4,63 g (30,8 mmol; 42%) eines allmählich kristallisierenden gelblichen Öls.

¹H-NMR (CDCl₃, 400 MHz) : δ = 10,63 (s, 1H; HC=0), 7,37 (t, 1H; *J* = 8,0 Hz; Ar-H), 6,80 (m, 2H; Ar-H), 3,88 (s, 3H; O-CH₃), 2,56 (s, 3H; Ar-CH₃). ¹³C-NMR (CDCl₃, 100,6 MHz): δ = 192,2 (C=O), 163,0 (Ar-C), 141,9 (Ar-C), 134,4 (Ar-CH), 123,9 (Ar-CH), 123,1 (Ar-C), 108,9 (Ar-CH), 55,6 (O-CH₃), 21,4 (CH₃).

### 2. Stufe: 2-Hydroxy-6-methylbenzaldehyd (HMBA)

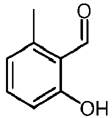

Eine Lösung von 2-Methoxy-6-methylbenzaldehyd (18,35 g, 0,122 mol) in Dichlormethan (150 ml) wurde vorsichtig mit wasserfreiem Aluminiumchlorid (24,44 g, 0,183 mol) versetzt und das zunächst heftig schäumende Reaktionsgemisch wurde bei Umgebungstemperatur gerührt. Nach 2 h wurde unter Eiskühlung Wasser (100 ml) zugetropft und die Phasen wurden getrennt. Die wässrige Phase wurde mit Dichlormethan (2x 50 ml) extrahiert. Die vereinigten organischen Phasen wurden mit gesättigter wässriger Natriumchlorid-Lösung (100 ml) gewaschen, über wasserfreiem Natriumsulfat getrocknet und über Kieselgel filtriert. Das Filtrat wurde am Rotationsverdampfer eingeengt. Man erhielt 14,9 g (0,110 mol; 90%) eines gelblichen Öls.

¹H-NMR (CDCl₂, 400 MHz) : δ = 11,90 (s, 1H; OH), 10,27 (s, 1H; HC=O), 7,34 (t, 1H; *J* = 8,0 Hz; Ar-H), 6,78 (d, 1H; *J* = 8,5 Hz, Ar-H), 6,68 (m, 1H; Ar-H), 2,56 (s, 3H; Ar-CH₃).

¹³C-NMR (CDCl₃, 100,6 MHz): δ = 195,2 (C=0), 163,0 (Ar-C), 142,0 (Ar-C), 137,2 (Ar-CH), 121,6 (Ar-CH), 118,3 (Ar-C), 115,9 (Ar-CH), 17,9 (CH₃).

### 3. Stufe: 4-[(2-Formyl-3-methylphenoxy)-methyl]-benzoesäure-methylester (FPBM)

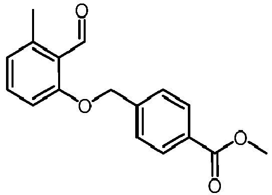

Eine Lösung von 2-Hydroxy-6-methylbenzaldehyd (23,52 g, 0,173 mol), 4-(Brommethyl)benzoesäuremethylester (39,57 g, 0,173 mol) und [18]Krone-6 (0,69 g, 2,6 mmol) in Aceton (250 ml) wurde mit Kaliumcarbonat (35,81 g, 0,259 mol) versetzt und das Gemisch wurde 24 h auf 40 °C erwärmt. Nach dem Abkühlen wurde die Suspension mit n-Heptan (200 ml) verdünnt und filtriert. Das Filtrat wurde am Rotationsverdampfer eingeengt. Der bräunliche Feststoff wurde in n-Heptan/Dichlormethan 1:2 (100 ml) gelöst und über Kieselgel filtriert. Das Filtrat wurde am Rotationsverdampfer eingeengt und man erhielt 46,48 g (0,163 mol; 94%) eines gelblichen Feststoffs.

¹H-NMR (CDCl₃, 400 MHz) : δ = 10,75 (s, 1H; HC=O), 8,06 (d, 2H; *J* = 8,0 Hz, Ar-H), 7,49 (d, 2H; *J* = 8,5 Hz, Ar-H), 7,35 (t, 1H; *J* = 8,0 Hz; Ar-H), 6,84 (m, 2H; Ar-H), 5,20 (s, 2H; O-CH₂), 3,91 (s, 3H; O-CH₃), 2,58 (s, 3H; Ar-CH₃).

¹³C-NMR (CDCl₃, 100,6 MHz) : δ = 191,8 (C=O), 166,5 (C=O), 161,8 (Ar-C), 142,1 (Ar-C), 141,2 (Ar-C), 134,3 (Ar-CH), 129,9 (Ar-CH), 129,8 (Ar-C), 126,7 (Ar-CH), 124,5 (Ar-CH), 123,4 (Ar-C), 110,2 (Ar-CH), 69,7 (O-CH₂), 52,1 (O-CH₃), 21,4 (CH₂).

### 4. Stufe: 4-[(2-Formyl-3-methylphenoxy)-methyl]-benzoesäure (FPBA)

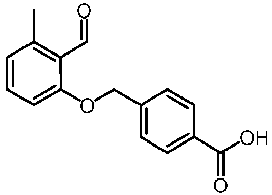

4-[(2-Formyl-3-methylphenoxy)-methyl]-benzoesäuremethylester (46,38 g, 0,163 mol) wurde in Dichlormethan (500 ml) gelöst und mit einer Lösung von Natriumhydroxid (19,58 g, 0,489 mol) in Methanol (150 ml) versetzt. Das Reaktionsgemisch wurde bei RT gerührt. Nach 24 h wurde die Suspension filtriert. Der Filtrationsrückstand wurde mit Dichlormethan (300 ml) gewaschen, in Wasser (300 ml) suspendiert und mit Salzsäure (2N; 250 ml) versetzt. Die Suspension wurde 1 h bei RT gerührt und filtriert. Der Filtrationsrückstand wurde mit Wasser (500 ml) gewaschen und getrocknet. Man erhielt 41,89 g (0,155 mol; 95%) eines leicht gelblichen Feststoffs.

¹H-NMR (Aceton-*d₆*, 400 MHz) : δ = 10,74 (s, 1H; HC=O), 8,09 (d, 2H; *J* = 8,1 Hz, Ar-H), 7,67 (d, 2H; *J* = 8,2 Hz, Ar-H), 7,46 (t, 1H; *J* = 8,2 Hz; Ar-H), 7,13 (d, 1H; *J* = 8,4 Hz; Ar-H), 6,89 (d, 1H; *J* = 8,0 Hz; Ar-H), 5,38 (s, 2H; O-CH₂), 2,53 (s, 3H; Ar-CH₃).

¹³C-NMR (Aceton-*d₆*, 100,6 MHz): δ = 191,6 (C=O), 167,0 (C=O), 162,3 (Ar-C), 142,2 (Ar-CH), 141,5 (Ar-C), 134,8 (Ar-CH), 130,7 (Ar-CH), 130,2 (Ar-CH), 127,6 (Ar-CH), 124,6 (Ar-CH), 123,9 (Ar-C), 111,3 (Ar-CH), 70,0 (O-CH₂), 20,8 (CH₃).

### 5. Stufe: 4-[(2-Formyl-3-methylphenoxy)-methyl]-benzoesäure-(2-methacryloyloxyethyl)-ester (PEMA)

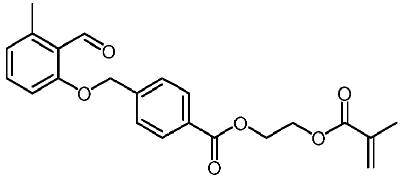

Ein Gemisch aus 4-[(2-Formyl-3-methylphenoxy)-methyl]-benzoesäure (43,77 g, 0,162 mol), Hydroxyethylmethacrylat (21,07 g, 0,162 mol), *N,N*-Dimethylaminopyridin (1,83 g, 15 mmol) und 2,6-Di-tert-butyl-4-methylphenol (25 mg) in Dichlormethan (500 ml) wurde auf 0 °C abgekühlt. *N,N'*-Dicyclohexylcarbodiimid (36,75 g, 0,178 mol) wurde portionsweise innerhalb von 20 min zugegeben. Das Reaktionsgemisch wurde 2 h unter Eiskühlung und anschließend bei Raumtemperatur gerührt. Nach 20 h wurde die Suspension mit n-Heptan (250 ml) verdünnt und über Kieselgel filtriert. Das Filtrat wurde am Rotationsverdampfer eingeengt. Das Rohprodukt wurde mittels Säulenchromatographie gereinigt (SiO₂, n-Heptan/Ethylacetat/Dichlormethan 4:1:2; R*_{f}* = 0,4). Man erhielt 49,98 g (0,130 mol; 81%) eines leicht gelblichen Feststoffs.

¹H-NMR (CDCl₃, 400 MHz) : δ = 10,75 (s, 1H; HC=O), 8,07 (d, 2H; *J* = 8,5 Hz, Ar-H), 7,51 (d, 2H; *J* = 8,5 Hz, Ar-H), 7,36 (t, 1H; *J* = 8,0 Hz; Ar-H), 6,85 (m, 2H; Ar-H), 6,15 (m, 1H; =CH), 5,59 (m, 1H; =CH), 5,22 (s, 2H; O-CH₂-Ar), 4,58 (m, 2H; O-CH₂-CH₂), 4,50 (m, 2H; O-CH₂-CH₂), 2,58 (s, 3H; Ar-CH₃), 1,95 (m, 3H; CH₂).

¹³C-NMR (CDCl₃, 100,6 MHz): δ = 191,8 (C=O), 167,0 (C=O), 165,8 (C=0), 161,7 (Ar-C), 142,1 (Ar-C), 141,5 (Ar-C), 135,8 (=C), 134,3 (Ar-CH), 130,0 (Ar-CH), 129,5 (Ar-C), 126,7 (Ar-CH), 126,0 (=CH₂), 124,6 (Ar-CH), 123,5 (Ar-C), 110,2 (Ar-CH), 69,7 (O-CH₂-Ar), 62,6 (O-CH₂-CH₂), 62,3 (O-CH₂-CH₂), 21,3 (Ar-CH₃), 18.2 (CH₃).

### Beispiel 2:

### 2-Stufige Photopolymerisation einer PEMA-haltigen Harzmischung

Ausgehend von dem Monomer PEMA aus Beispiel 1 wurde mit den Comonomeren Butylmethacrylat (BMA) und Methylmethacrylat (MMA) Monomermischungen hergestellt, denen jeweils 50 mmol/l Bis(2,4,6-trimethylbenzoyl)phenylphosphinoxid (Irgacure® 819, Fa. Ivoclar) zugesetzt wurde (Tab. 1). Die Mischungen wurden zunächst mit einer blauen LED (λ = 445-465 nm, Intensität: 7 mW/cm²) für 1 h bestrahlt. Anschließend wurden die Mischungen für unterschiedliche Zeiten in einem Photoreaktor (Modell LZC-4V, Luzchem Research Inc., Ottawa, Kanada) mit einer UV-A-Strahlungsquelle (λₘₐₓ = 350 nm) mit einer Intensität von 5 mW/cm², bei einer Temperatur von ungefähr 45°C bestrahlt. Die mechanischen Eigenschaften (reduzierter E-Modul Eᵣ und Härte) der ausgehärten Proben wurden mittels Nanoindentation (instrumentierter Eindringversuch) unter Verwendung eines Hysitron TL950 Nanoindentor (Bruker) bestimmt (vgl. Tab. 1). Die ladungskontrollierten Messungen mit einer Berkovich-Spitze wurden bei 2000 µN maximaler Ladekraft mit einer Ladezeit von 1 s, einem Ladesegment von 2 s sowie einer Lastwegnahme von 1 s durchgeführt. Die Analyse erfolgte unter Verwendung der Oliver- und Pharr-Methode (W. C. Oliver, G.M. Pharr, J. Mater In: Res. 7, 1992, S. 1564). Es wurde mit Messmethoden analog zu B. Sandmann, B. Happ, J. Vitz, R. M. Paulus, M. D. Hager, P. Burtscher, N. Moszner, U. S. Schubert, Macromol. Chem. Phys. 2014, 215, 1603.) gemessen.

### Tabelle 1: Zusammensetzungen (mol-%) von Harzen mit BMA, MMA und PEMA und mechanische Eigenschaften der ausgehärteten Proben

| BMA^{a} [%] | MMA^{b} [%] | PEMA^{c} [%] | t_{cure} [min] | Eᵣ [GPa] | Härte [MPa] |
|---|---|---|---|---|---|
| 30 | 55 | 15 | 0 | 2,97 ± 0,49 | 34,3 ± 3,9 |
| 30 | 55 | 15 | 1 | 4,58 ± 0,02 | 165,3 ± 1,8 |
| 30 | 55 | 15 | 2 | 5,1 ± 0,02 | 263,8 ± 3,6 |
| 30 | 55 | 15 | 5 | 5,27 ± 0,03 | 294,3 ± 5,7 |

| | | | | | |
|---|---|---|---|---|---|
| a = Butylmethacrylat b = Methylmethacrylat c = 4-[(2-Formyl-3-methylphenoxy)-methyl]-benzoesäure-(2-methacryloyloxyethyl)-ester (Bsp. 1) | | | | | |

Die Ergebnisse in Tab. 1 belegen, dass mit zunehmender UV-A-Bestrahlung der E-Modul und die Härte der Prüfkörper zunehmen, was durch eine Dimerisierung der photochemisch-induzierten o-Quinodimethan-Bausteine hervorgerufen wird. Im Vergleich dazu liegen die Referenzwerte für eine kommerziell erhältliche Zahnspange aus Polyurethan (Invisalign Aligner, Firma Align Technology BV) bei Eᵣ =0,95 ± 0,09 GPa bzw. 45 ± 1,7 MPa.

### Beispiel 3:

### Simultane Photopolymerisation von PEMA-haltigen Harzmischungen

Ausgehend vom Monomer PEMA aus Beispiel 1 wurden mit den Comonomeren BMA, MMA und/oder Methylacrylat (MA) Monomermischungen hergestellt, denen 50 mmol/l Bis(2,4,6-trimethylbenzoyl)-phenylphosphinoxid (Irgacure® 819, Fa. Ivoclar) zugesetzt wurde (Tab. 2). Die Harze wurden in einem Photoreaktor (Modell LZC-4V, Luzchem Research Inc., Ottawa, Kanada) mit einer UV-A-Strahlungsquelle (λₘₐₓ = 350 nm) mit einer Intensität von 5 mW/cm⁻² bei einer Temperatur von 45 bis 50 °C bestrahlt. Mechanischen Eigenschaften (E-Modul Eᵣ und Härte) der ausgehärten Proben wurden wieder mittels Nanoindentation wie in Beispiel 2 beschrieben bestimmt (Tab. 2).

**Tabelle 2: Harzzusammensetzungen (mol-%) und mechanische Eigenschaften der ausgehärteten Proben**

| Probe | BMA^{a} [%] | MMA^{b} [%] | MA^{c} [%] | PEMA^{d} [%] | *t*_{c}^{e} [min] | Eᵣ^{f} [GPa] | Härte [MPa] |
|---|---|---|---|---|---|---|---|
| A | 34 | 61 | / | 5 | 60 | 3,85 ± 0,07 | 109,7 ± 1,7 |
| B | 32 | 58 | / | 10 | 60 | 5,43 ± 0,11 | 350 ± 19,8 |
| C | / | / | 97 | 3 | 30 | 0,29 ± 0,04 | 52,0 ± 11,9 |
| D | / | / | 96 | 4 | 30 | 0,89 ± 0,03 | 89,6 ± 7,4 |
| E | / | / | 95 | 5 | 30 | 1,26 ± 0,22 | 93,9 ± 17,7 |
| F | / | / | 90 | 10 | 30 | 5,76 ± 0,06 | 329,3 ± 1,7 |

| | | | | | | | |
|---|---|---|---|---|---|---|---|
| a = Butylmethacrylat b = Methylmethacrylat c = Methylacrylat d = 4-[(2-Formyl-3-methylphenoxy)-methyl]-benzoesäure-(2-methacryloyloxyethyl)-ester (Bsp. 1) e = Härtungszeit f = Reduzierter E-Modul | | | | | | | |

Die Ergebnisse belegen, dass in Abhängigkeit von der Harzzusammensetzung Polymernetzwerke mit einstellbarer Flexibilität hergestellt werden können. Mit steigendem Anteil von PEMA nehmen E-Modul und Härte der Proben zu.

### Beispiel 4:

### Radikalische Polymerisation und Dimerisierung von photochemisch-induzierten o-Quinodimethan-Bausteinen

Die Polymernetzwerksynthese erfolgte in zwei Stufen: 1. Radikalische Copolymerisation des photoenolisierbaren Monomer PEMA mittels eines thermischen Initiators und 2. Bestrahlung mit UV-A-Licht zur Dimerisierung von photochemisch-induzierten o-Quinodimethan-Bausteinen. Zunächst wurden Monomermischungen (Tab.3) hergestellt und dazu jeweils 50 ml Toluol als Lösungsmittel, 2 mol-% Azobisisobutyronitril (AIBN) als thermischer Initiator und 2 mol-% Laurylmercaptan als Kettenregler zugegeben. Nach dem Entgasen der Monomerlösungen wurden diese bei 65°C (Probe b: 75 °C) polymerisiert, die Polymerisate aus wässrigem Methanol (10 % Wasser) ausgefällt und im Feinvakuum getrocknet. Die Bestimmung der zahlenmittleren Molmasse Mₙ und des Polydispersitätsindexes (PDI) der erhaltenen Copolymeren erfolgte mittels Gelpermeationschromatographie (GPC) unter Verwendung eines PSS SECurity-Systems (Polymer Standards Service GmbH, Mainz), während die Glasübergangstemperaturen T_{g} mittels Differential-Scanning-Calorimetrie (DSC) mit dem Gerät Q100 SDC V9.6 Build 290 (TA Instruments) bestimmt wurden (Tab. 4). Die DSC-Messungen erfolgten zwischen -40 und 120 °C mit einer Heiz- bzw. Kühlrate von 10 °C/min. Es wurden jeweils zwei Heiz- und Kühlzyklen aufgenommen, wobei die aufgeführten Glasübergangstemperaturen dem zweiten Heizsegment entnommen sind.

**Tabelle 3: Monomerzusammensetzungen (mol-%) für die radikalische Copolymerisation**

| Probe | BMA ^{a} | | MMA ^{b} | | PEMA ^{c} | | THFMA ^{d} | | IBOMA ^{e} | |
|---|---|---|---|---|---|---|---|---|---|---|
| | [%] | mmol | [%] | mmol | [%] | mmol | [%] | mmol | [%] | mmol |
| A | - | - | 77 | 24,8 | 23 | 6,04 | - | - | - | - |
| B | 34 | 9,67 | 46 | 15,65 | 20 | 6,23 | - | - | - | - |
| C | 65 | 20,38 | 15 | 4,70 | 20 | 6,20 | - | - | - | - |
| D | - | - | - | - | 24 | 1,66 | 76 | 6,72 | - | - |
| E | - | - | - | - | 20 | 1,80 | - | - | 80 | 7,25 |

| | | | | | | | | | | |
|---|---|---|---|---|---|---|---|---|---|---|
| a = Butylmethacrylat b = Methylmethacrylat c = 4-[(2-Formyl-3-methylphenoxy)-methyl]-benzoesäure-(2-methacryloyloxyethyl)-ester d = Tetrahydrofurfurylmethacrylat e = Isobornylmethacrylat | | | | | | | | | | |

**Tabelle 4: GPC- und DSC-Ergebnisse der Copolymere**

| Probe | BMA^{a} [%] | MMA^{b} [%] | PEMA^{c} [%] | THFMA^{d} [%] | IBOMA^{e} [%] | tₚ^{f} [h] | Mₙ [g/mol⁻¹] | PDI | T_{g} [°C] |
|---|---|---|---|---|---|---|---|---|---|
| A | / | 77 | 23 | / | / | 24 | 6800 | 1,8 | 65 |
| B | 34 | 46 | 20 | / | / | 4 | 10000 | 1,7 | 51 |
| C | 65 | 15 | 20 | / | / | 21 | 7000 | 1,7 | 23 |
| D | / | / | 24 | 76 | / | 22 | 5700 | 1,9 | 40^{g} |
| E | / | / | 20 | / | 80 | 22 | 6600 | 1,4 | 116^{g} |

| | | | | | | | | | |
|---|---|---|---|---|---|---|---|---|---|
| a = Butylmethacrylat b = Methylmethacrylat c = 4-[(2-Formyl-3-methylphenoxy)-methyl]-benzoesäure-(2-methacryloyloxyethyl)-ester (Bsp. 1) d = Tetrahydrofurfurylmethacrylat e = Isobornylmethacrylat f = tₚ = Polymerisationszeit g = hier wurden die DSC-Messungen im Temperaturbereich bis 140 °C durchgeführt | | | | | | | | | |

Die Ergebnisse der geregelten Copolymerisationsationen ergaben Copolymere mit einer zahlenmittleren Molmasse zwischen 6800 und 10000 g/mol und einer Glasübergangstemperatur zwischen 40 und 116 °C. Die Copolymere A bis E sind bei 140 °C formbar. Es wurden Filme und Presslinge hergestellt, die sich nach Bestrahlung mit UV-Licht (365 nm) für 2 min als unlöslich erwiesen, was durch eine Dimerisierung der photochemisch-induzierten o-Quinodimethan-Bausteine hervorgerufen wird.

### Beispiel 5:

### Simultane Photopolymerisation von o-Methylbenzaldehyd-Methacrylaten- und MA-haltigen Harzmischungen

Mit dem Monomer PEMA aus Beispiel 1 wurden Monomermischungen mit dem Comonomer MA (Proben a-c) oder MA und einem flüssigen 2-Ureido-4[1H]-pyrimidinonmethacrylamid (UPyMAA, Probe d), das nach der Literatur (Heinzmann et al., Macromolecules 48 (2015) 8128-8136) synthetisiert wurde, hergestellt und 2 mol-% Bis(2,4,6-trimethylbenzoyl)phenylphosphinoxid (Irgacure® 819, Fa. Ivoclar) zugesetzt (Tab. 5). Die Harze wurden jeweils in Aluminiumschalen innerhalb eines Photoreaktors (Modell LZC-4V, Luzchem Research Ic. Ottawa, Kanada) mit einer UV-A-Strahlungsquelle (λₘₐₓ = 350 nm) mit einer Intensität von 5 mW/cm⁻² bestrahlt. Die Temperatur war Raumtemperatur (RT) oder 50°C. Die mechanischen Eigenschaften (E-Modul und Härte) der ausgehärten Proben wurden wieder mittels Nanoindentation wie in Beispiel 2 beschrieben bestimmt (Tab. 5).

### Tabelle 5: Zusammensetzung (mol-%) von Harzen mit PEMA und MA und mechanische Eigenschaften der ausgehärteten Proben

| Probe | UPyMAA^{a} (%) | PEMA^{b} (%) | MA^{c} (%) | T_{c}^{d} (°C) | t_{c}^{e} (min) | Eᵣ (GPa) | Härte (MPa) |
|---|---|---|---|---|---|---|---|
| A | / | 15 | 85 | 50 | 30 | 5,37 ± 0,32 | 328,2 ± 12,1 |
| B | / | 20 | 80 | 50 | 30 | 5,23 ± 0,04 | 301,9 ± 1,9 |
| C | / | 20 | 80 | 50 | 45 | 5,49 ± 0,03 | 303,2 ± 1,4 |
| D | 10 | 10 | 80 | 50 | 45 | 3,51 ± 0,01 | 123,5 ± 1,5 |

| | | | | | | | |
|---|---|---|---|---|---|---|---|
| a = 2-Ureido-4[1H]-pyrimidinonmethacrylamid b = 4-[(2-Formyl-3-methylphenoxy)-methyl]-benzoesäure-(2-methacryloyloxyethyl)-ester c = Methylacrylat d = Härtungstemperatur e = Härtungszeit | | | | | | | |

Die Ergebnisse belegen, dass mit wechselndem Anteil von erfindungsgemäßem Vernetzer (PEMA) Polymernetzwerke mit sehr unterschiedlicher Härte und E-Modul hergestellt werden können. Dabei liegen die erhaltenen Werte in einem Bereich, der für Korrekturschienen typisch ist.

## Patentansprüche

1. Zusammensetzung, die mindestens ein polymerisierbares Benzaldehyd-Derivat gemäß der allgemeinen Formel I enthält, 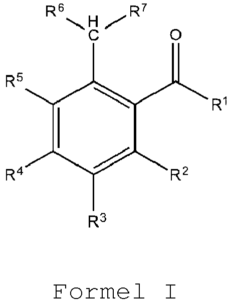 in der
R¹ Wasserstoff, eine verzweigte oder unverzweigte, gesättigte oder ungesättigte C₁-C₁₄-Alkylgruppe, die durch O oder S unterbrochen sein kann, eine C₆-C₁₄-Arylgruppe oder eine C₄-C₁₄-Heteroarylgruppe ist, die N, O oder S enthalten kann, wobei die Alkyl-, Aryl- oder Heteroarylgruppen mit Thioether-, Amino-, Alkoxy- oder Alkylgruppen mit 1 bis 14 Kohlenstoffatomen oder C₆-C₁₄-Arylgruppen substituiert sein können und wobei die Substituenten miteinander verbrückt sein können,
R²⁻⁵ jeweils unabhängig voneinander Wasserstoff, eine NH₂-Gruppe, eine verzweigte oder unverzweigte, gesättigte oder ungesättigte Thioether-, Amino-, Alkoxy- oder Alkylgruppe mit 1 bis 14 Kohlenstoffatomen, eine verzweigte oder unverzweigte, gesättigte oder ungesättigte C₇-C₁₅-Arylalkoxygruppe, vorzugsweise Benzyloxygruppe (Ph-CH₂-O-), oder eine C₆-C₁₄-Arylgruppe sind, die über O oder S angebunden sein kann, wobei R²⁻⁵ miteinander verbrückt sein können und R² und/oder R⁴ -X-SP-PG sind/ist,
X entfällt oder -O-, -CO-O- oder -O-CO- ist,
SP eine lineare oder verzweigte C₁-C₆-Alkylen-Gruppe ist, wobei die Alkylen-Gruppe durch O, S, -CO-O-, -O-CO- und/oder Phenylen unterbrochen sein kann,
PG eine polymerisierbare Gruppe ist,
R⁶⁻⁷ jeweils unabhängig voneinander Wasserstoff oder eine C₁-C₁₀-Alkylgruppe sind, die verzweigt oder unverzweigt, gesättigt oder ungesättigt sein kann.

2. Zusammensetzung nach Anspruch 1, wobei die Variablen die folgenden Bedeutungen haben:
R¹ Wasserstoff oder C₁-C₄-Alkylgruppe,
R²⁻⁵ jeweils unabhängig voneinander Wasserstoff oder eine C₁-C₄-Alkoxy- oder C₁-C₄-Alkylgruppe, wobei R² und/oder R⁴ -X-SP-PG sind/ist,
X -O-,
SP eine lineare C₂-C₆-Alkylen-Gruppe, wobei die Alkylen-Gruppe durch O, -CO-O- und/oder Phenylen unterbrochen sein kann, vorzugsweise eine C₂-C₄-Alkylen-Gruppe oder -CH₂-Ph-CO-O-CH₂CH₂-,
PG eine kationisch oder vorzugsweise radikalisch polymerisierbare Gruppe, insbesondere eine (Meth)acrylat-Gruppe oder eine Acrylamid-Gruppe,
R⁶⁻⁷ beide Wasserstoff oder R⁶ = C₁-C₁₀-Alkyl und R⁷ = H.

3. Zusammensetzung nach Anspruch 1, wobei die Variablen die folgenden Bedeutungen haben:
R¹ Wasserstoff oder -CH₃,
R²⁻⁵ jeweils unabhängig voneinander Wasserstoff, -CH₃ oder -OCH₃, wobei R² oder R⁴ -X-SP-PG ist,
X -O-,
SP eine lineare C₂-C₆-Alkylen-Gruppe, wobei die Alkylen-Gruppe durch O, -CO-O- und/oder Phenylen unterbrochen sein kann, vorzugsweise eine C₂-C₄-Alkylen-Gruppe oder -CH₂-Ph-CO-O-CH₂CH₂-,
PG eine kationisch oder vorzugsweise radikalisch polymerisierbare Gruppe, insbesondere eine (Meth)acrylat-Gruppe oder eine Acrylamid-Gruppe,
R⁶⁻⁷ jeweils Wasserstoff.

4. Zusammensetzung nach Anspruch 1, wobei die Variablen die folgenden Bedeutungen haben:
R¹ Wasserstoff,
R²⁻⁵ jeweils unabhängig voneinander Wasserstoff oder -CH₃, wobei R² oder R⁴ -X-SP-PG ist,
X -O-,
SP eine lineare C₂-C₄-Alkylen-Gruppe, wobei die Alkylen-Gruppe durch O, -CO-O- und/oder Phenylen unterbrochen sein kann, vorzugsweise eine C₂-Alkylen-Gruppe oder -CH₂-Ph-CO-O-CH₂CH₂-,
PG eine radikalisch polymerisierbare Gruppe, insbesondere eine (Meth)acrylat-Gruppe oder eine Acrylamid-Gruppe,
R⁶⁻⁷ jeweils Wasserstoff.

5. Zusammensetzung nach einem der Ansprüche 1 bis 4, in der PG eine radikalisch polymerisierbare Gruppe ist, die aus einer Vinyl-, (Meth)acrylat- oder (Meth)acrylamid-Gruppe ausgewählt ist.

6. Zusammensetzung nach einem der Ansprüche 1 bis 4, in der PG eine kationisch polymerisierbare Gruppen ist, die aus einer Epoxid-, Oxetan- oder Vinylether-Gruppe ausgewählt ist.

7. Zusammensetzung nach einem der Ansprüche 1 bis 6, die keine weiteren 1,3-Diene und keine Dienophile enthält, die Cycloadditionsreaktionen eingehen.

8. Zusammensetzung nach einem der Ansprüche 1 bis 7, die zusätzlich mindestens ein weiteres polymerisierbares Monomer, vorzugsweise mindestens ein radikalisch polymerisierbares mono- oder multifunktionelles (Meth)acrylat, besonders bevorzugt ein Methacrylat enthält.

9. Zusammensetzung nach Anspruch 8, die als zusätzliches Monomer Methylacrylat (MA), Ethylacrylat (EA),Methylmethacrylat (MMA), Butylmethacrylat (BMA), Tetrahydrofurfurylmethacrylat (THFMA), Isobornylmethacrylat (IBOMA) oder eine Mischung davon enthält.

10. Zusammensetzung nach einem der Ansprüche 1 bis 9, die zusätzlich mindestens einen Initiator für die Polymerisation durch UV-A-Licht, sichtbares Licht, einen thermischen Initiator und/oder einen Redoxinitiator enthält.

11. Zusammensetzung nach einem der Ansprüche 1 bis 10, die zusätzlich mindestens einen anorganischen Füllstoff enthält.

12. Zusammensetzung nach einem der Ansprüche 1 bis 11, die
a) 0,1 bis 50 Gew.-%, bevorzugt 1 bis 40 Gew.-% und besonders bevorzugt 1 bis 20 Gew.-% mindestens eines Benzaldehyd-Derivats der Formel I,
b) 30 bis 99 Gew.-%, bevorzugt 40 bis 95 Gew.-% und besonders bevorzugt 50 bis 95 Gew.-% mindestens eines weiteren monofunktionellen radikalisch oder kationisch polymerisierbaren Monomers,
c) 0 bis 20 Gew.-%, bevorzugt 0 bis 15 Gew.-% und besonders bevorzugt 0 bis 10 Gew.-% mindestens eines multifunktionellen radikalisch oder kationisch polymerisierbaren Monomers,
d) 0,001 bis 5,0 Gew.-%, bevorzugt 0,01 bis 3,0 Gew.-% und besonders bevorzugt 0,1 bis 1,0 Gew.-% mindestens eines Initiators, insbesondere eines Photoinitiators,
e) 0 bis 5,0 Gew.-%, bevorzugt 0 bis 3,0 Gew.-% und besonders bevorzugt 0 bis 1,5 Gew.-% Additiv(e) enthält,
jeweils bezogen auf die Gesamtmasse des Werkstoffs.

13. Verwendung einer Zusammensetzung nach einem der Ansprüche 1 bis 12 als medizintechnischer Werkstoff zur Herstellung von Medizinprodukten, Implantaten für Gehörprothesen, Knorpel-oder Knochenersatzteilen, als Werkstoff für die Augenheilkunde, zur Herstellung von Intraokularlinsen.

14. Verwendung einer Zusammensetzung gemäß einem der Ansprüche 1 bis 12 zur extraoralen Herstellung oder Reparatur von Zahnspangen, Korrekturschienen oder Schablonen zur Korrektur von Zahnfehlstellungen.

15. Verwendung eines Benzaldehyd-Derivats gemäß Formel I zur Herstellung eines medizintechnischen Werkstoffs.
